(19) Europäisches Patentamt
European Patent Office
Office européen des brevets

(11) **EP 4 181 767 B1**

(12) **EUROPEAN PATENT SPECIFICATION**

(45) Date of publication and mention
of the grant of the patent:
**20.08.2025 Bulletin 2025/34**

(21) Application number: **21746815.6**

(22) Date of filing: **08.07.2021**

(51) International Patent Classification (IPC):
***A61B 3/16*** (2006.01)

(52) Cooperative Patent Classification (CPC):
**A61B 3/16**

(86) International application number:
**PCT/IB2021/056141**

(87) International publication number:
**WO 2022/013689 (20.01.2022 Gazette 2022/03)**

(54) **DISPOSABLE APPLANATION HEAD FOR AN APPLANATION TONOMETER**

EINWEG-APPLANATIONSKOPF FÜR EINEN APPLANATIONSTONOMETER

TÊTE D'APLANISSEMENT JETABLE POUR TONOMÈTRE À APLANATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.07.2020 IT 202000017059**

(43) Date of publication of application:
**24.05.2023 Bulletin 2023/21**

(73) Proprietor: **Frastema Ophthalmics Easyopht
Group S.r.l.**
**(in forma abbreviata FE - Group S.r.l.)**
**21058 Solbiate Olona VA (IT)**

(72) Inventor: **CARNAGHI, Matteo**
**21052 Busto Arsizio VA (IT)**

(74) Representative: **Barbaro, Gaetano et al
Società Italiana Brevetti S.p.A.
Via G. Carducci, 8
20123 Milano (IT)**

(56) References cited:
**EP-A2- 3 260 041      WO-A1-2013/132396
US-A- 3 301 131        US-A1- 2017 215 728**

**Description**

TECHNICAL FIELD

This disclosure relates to tonometers for measuring intraocular pressure and more particularly to a head for a Goldmann applanation tonometer and a related Goldmann tonometer for measuring the intraocular pressure of a human or animal eye, as well as a disposable kit for measuring intraocular pressure.

BACKGROUND OF THE INVENTION

The measurement of intraocular pressure (IOP: IntraOcular Pressure) represents a fundamental moment of the routine ophthalmological examination, as the increase of its value represents a major risk factor for glaucoma and is one of the fundamental parameters for the diagnosis of this disease, together with the evaluation of the optic disc and the examination of the visual field.

Tonometry is a procedure that allows an indirect measurement of IOP, based on the relationship between intraocular pressure and the force necessary to modify the natural shape of the cornea, using instruments called tonometers.

In the following, reference will only be made to the "applanation" tonometers, to which the invention disclosed herein applies. They measure the force required to flatten a surface having a known area of the cornea or evaluate the width of the area flattened by a predetermined fixed force. In particular, Goldmann's applanation tonometer, now universally used, represents the international standard for measuring IOP.

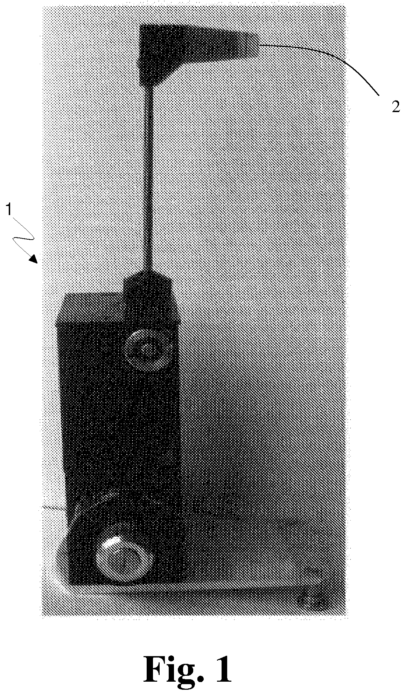

The Goldmann tonometer, depicted in Figure 1, includes a flattening element, or head, reusable or disposable, consisting of a typically transparent plastic head intended to come into contact with the patient's eye. The head of the tonometer, containing one or more prisms, is joined by means of a rod to a spiral spring. By means of a lateral graduated knob it is possible to vary the value of the force applied to the cornea by means of the head of the tonometer. EP 3 260 041 discloses such a tonometer.

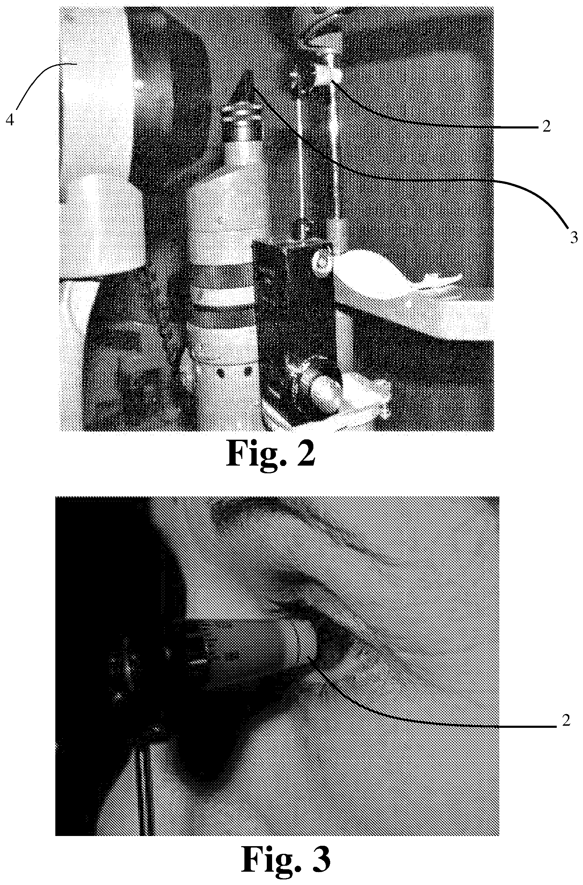

The examination technique involves placing the instrument on a special base of a slit lamp, shown in figure 2. After corneal anesthesia, the folded end of a strip of filter paper soaked in ophthalmic dye, a fluorescent substance that serves to make the edge of the smoothed area more evident by observation with blue light, is applied to the patient's lower conjunctival fornix. A widely used ophthalmic dye is fluorescein, which emits an intense yellow-green fluorescence in the 520-570 nm wavelength range when excited by blue light radiation in the 450-500 nm range (or by ultraviolet radiation in the range 250-400 nm). As an alternative to fluorescein, riboflavin is used, which emits yellow-green fluorescence in the 520-570 nm range when excited by blue light radiation in the 450-500 nm range (or ultraviolet radiation in the 250-400 nm range). Identifying the edges of the flattened area with good precision is essential for a correct measurement of ocular pressure.

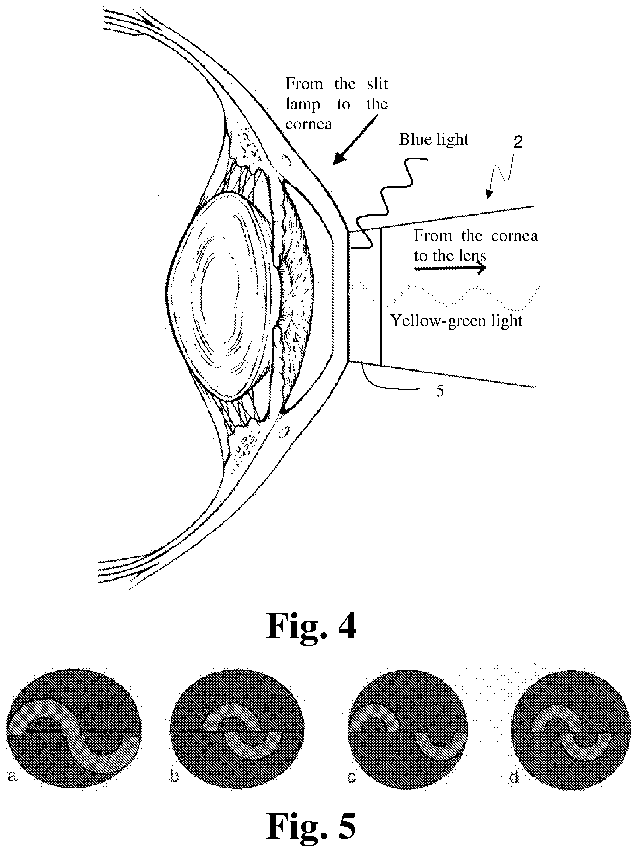

The patient is seated in front of the slit lamp and asked to observe a reference point, as shown in figure 3. The slit of the lamp is opened to the maximum and a cobalt blue filter is placed in front of it to better visualize the ophthalmic dye, that is, to better visualize the edges of the smoothed area. A light beam is then directed so that it passes through the transparent prism and the knob is adjusted to the notch corresponding to 10 mmHg. The slit lamp is moved forward so that the prism gently contacts the central part of the cornea, as in figure 4.

The prism, contained in the transparent plastic head, splits the circular image of the flattened corneal surface into two semi-annuli, one on top of the other. Then through the eyepiece, you can see two green semi-annuli in a blue luminous field.

Figure 5 shows what is observed with the Goldmann tonometer in different cases. The side knob of the tonometer is rotated in such a way as to bring the two semi-annuli into contact with their inner edge. Depending on the pressure exerted, different configurations of the semi-annuli can be observed: if they are far away it is necessary to increase the applanation force, if they overlap it is necessary to rotate the knob in reverse to decrease the exerted force. In Figure 5A, the semi-annuli appear relatively thick due to excessive use of ophthalmic dye; in figure 5B the semi-annuli have an adequate thickness but are not correctly aligned because the applanation force is excessive; in figure 5C, the applanation force is insufficient; in figure 5D the semi-annuli are correctly aligned.

The ability to correctly identify the two semi-annuli, their respective ends and their respective internal edges is a crucial factor for the correct calibration of the instrument and it is not easy for the physician to completely discriminate the two green semi-annuli within the predominant blue luminous field.

A solution to facilitate the visualization of the semi-annuli involves the insertion of a yellow filter-barrier above the eyepiece of the slit lamp. It filters out blue light to make the fluorescent green stand out more clearly. Filters available on the market are characterized by transparent yellow lenses, with a reduced thickness, about 80$\mu$m. However, the use of a filter with a reduced thickness, in addition to requiring the purchase of an additional component (the filter itself), constitutes the only element for filtering the intense light radiation coming from the patient's eye, sometimes making it difficult, for the physician, the distinction of the thin green semi-annuli immersed in a field of blue light and therefore does not constitute an optimal solution.

It would be desirable to improve the filtering of blue light from the patient's eye during a tonometry measurement and eliminate the need to purchase an additional component such as a yellow barrier filter for the slit lamp used during this measurement test.

## SUMMARY OF THE INVENTION

The subject of this disclosure is an applanation head for a Goldmann applanation tonometer, as well as a related Goldmann applanation tonometer, with integrated yellow filter-barrier coloring.

The addressed problem is solved by means of a Goldmann applanation tonometer head according to claim 1.

Preferred features of the invention are the subject of the dependent claims.

A disposable kit for intraocular pressure measurement with a Goldmann applanation tonometer is also disclosed, comprising a package containing a Goldmann applanation tonometer head according to this disclosure, and a single-dose bottle filled with a liquid dye for ophthalmic use or a paper strip soaked with a liquid or powder dye for ophthalmic use.

## BRIEF DESCRIPTION OF THE DRAWINGS

Other advantages and characteristics of the present invention will become evident from the following detailed description of some embodiments, presented by way of nonlimiting example with reference to the attached drawings, in which:

    Figure 1 shows a Goldmann tonometer;
    Figure 2 shows the Goldmann tonometer of Figure 1 mounted on a base of a slit lamp;
    Figure 3 shows the Goldmann tonometer head applied to a patient's eye;
    Figure 4 illustrates the principle of measuring intraocular pressure with the Goldmann tonometer;
    figure 5 shows semi-annuli visualized with the Goldmann tonometer, in the following cases: a) excessive use of ophthalmic dye; b) excessive applanation force; c) insufficient applanation force; d) semi-annuli correctly aligned;
    Figure 6 shows an applanation head for a Goldmann tonometer according to the present disclosure;
    Figure 7 shows the spectrophotometric characterization of an applanation head for a Goldmann tonometer according to the present disclosure;
    Figure 8 is a diagram of the color reference system illustrating the meaning of the parameters used to characterize the yellow colors that can be used to make a head for a tonometer according to the present disclosure; is
    Figure 9 shows a table showing the numerical values of the classification parameters of the yellow hue of the applanation head according to the present disclosure.

## DETAILED DESCRIPTION

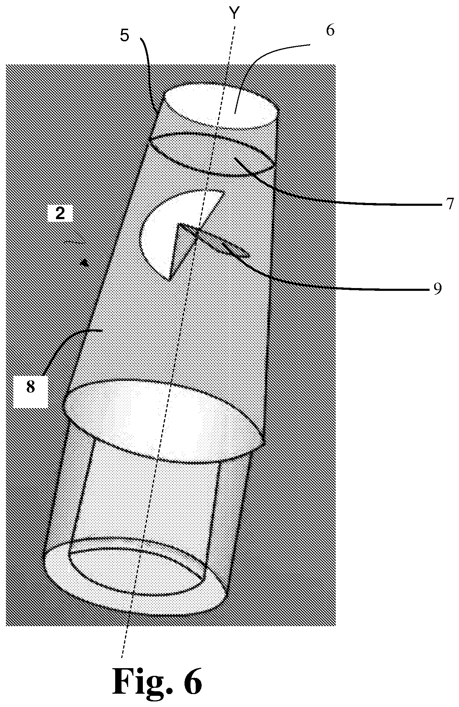

An exemplary embodiment of an applanation head 2 for a Goldmann tonometer 1 according to this disclosure is schematically illustrated in figure 6. In the following reference will be made to this embodiment, which is preferred, although several other embodiments are possible.

The head 2 for Goldmann applanation tonometer 1 according to this disclosure is made of a transparent polymeric material, for example in Polymethylmethacrylate (PMMA) and defines:

- a body 8 having a flat and transparent applanation surface 6, with a circular profile, intended to come into contact with the cornea of a patient's eye and configured to be pressed against it so as to flatten it. Said body 8 defines a longitudinal development axis Y perpendicular to said applanation surface 6, as in figure 6;
- an optical part comprising two optical prisms 9, visible in transparency in figure 6 and positioned so that their center is crossed by said longitudinal axis Y, configured to split the image of the circular footprint into two semi-annuli, as in figure 5, over a patient's cornea during an applanation intraocular pressure measurement.

Studies carried out by the applicant have shown that, by making the head for tonometer in a transparent yellow material, with a carefully determined hue and saturation, it is possible to avoid the use of the common yellow barrier filters, guaranteeing an effective filtering of the blue light radiation through the optical path of the tonometer head, making the semi-annuli more visible during the measurement of intraocular pressure with applanation tonometry.

Unlike the common applanation heads for Goldmann tonometers, the applanation head 2 according to this disclosure has at least one portion 5 characterized by a transparent yellow color. Said at least one portion 5 comprises the applanation surface 6 and defines optical prisms 9 configured to visualize as two circular semi-annuli a substantially circular perimeter of a cornea flattened against the flat corneal applanation surface 6. According to this disclosure, the coloring of this transparent portion is of a different intensity of yellow than common barrier filters, as will be described below.

The transparent yellow color is obtained by adding a dye during the polymerization phase of said transparent polymer in correspondence with said at least one portion 5, in a quantity ranging from 0.5 to 1.5% (w / w). According to a preferred embodiment of the present invention, the dye used is of the type currently known under the trade name RENOL™ yellow produced by Clariant, added at 1% (w / w) in the polymerization phase of PMMA. Preferably, this yellow dye is selected from the set consisting of yellow dyes suitable for PMMA coloring. According to one aspect, a suitable yellow dye may be of the type provided in concentrated form in a masterbatch using a carrier composed of PMMA (polymethyl methacrylate) or SAN (styrene acrylonitrile).

An applanation head 2 according to the present disclosure is characterized by a macroscopically lighter yellow color than the common commercially available tonometry filters. In fact, with a reduced thickness, generally 80μm, the filtering action of common filters is mainly due to the filtering power of an intense yellow hue.

Making an applanation head 2 with the same coloration as common barrier filters would not be optimal for the tonometer measurement, since the blue excitation light, coming from the slit lamp 3 and directed to the patient's cornea, passes always through a terminal portion of the applanation head of the tonometer, in a diagonal direction with respect to said Y axis, as shown in figure 4. The attenuation of the impinging blue light could limit the excitation, or not excite at all, of the ophthalmic dye on the cornea and make measurement impracticable.

Consequently, it is desirable that the coloring of said portion 5 of the applanation head 2 according to this disclosure be relatively light.

This shade, following the experimental confirmation made by the Applicant, allows an optimal passage of the blue radiation of excitation of the ophthalmic dye so that the latter is excited and emits fluorescence in the yellow spectrum in an optimal manner for the success of the measurement.

A filter with a thickness similar to that of common barrier filters, about 80μm, and with the attenuated yellow color described above would not be efficient in filtering the blue light reflected from the cornea and directed to the eyepiece 4 of the slit lamp 3, for obvious reasons. However, the blue light intended to excite the ophthalmic dye only passes through a limited terminal portion of the applanation head 2, with a diagonal incidence with respect to the Y axis. The reflected blue light, on the other hand, travels the entire optical path of the applanation head 2, in direction parallel to the Y axis, crossing a larger volume of the same. As a result, the blue light reflected from the cornea is attenuated more than the blue light directed from the slit lamp towards the cornea. The Applicant noted that, thanks to an appropriate calibration of the yellow color of the transparent body 8, the semi-annuli stand out more against the blue background of the light reflected by the cornea, allowing a more accurate detection of the patient's intraocular pressure.

The criteria used by the Applicant for the characterization of the yellow color are indicated below.

To characterize the yellow color of the transparent mixture that constitutes the transparent body 8 of the tonometer head, reference was made to the ASTM E313 standard in force in 2019.

According to this standard, the classification of the yellow color is based on the L * a * b colorimetric model defined by the International Commission for Illumination (CIE), also known as CIELab, in which a color is indicated by three values:

L, Luminance parameter, expressed as a percentage, in which a value L = 0 indicates black and a value L = 100 indicates white;

a, dimensionless parameter relating to a color range for which a value a = -100 indicates green and a value a = +100 indicates red;

b, dimensionless parameter relating to a color range for which a value b = -100 indicates blue and a value b = +100 indicates yellow;

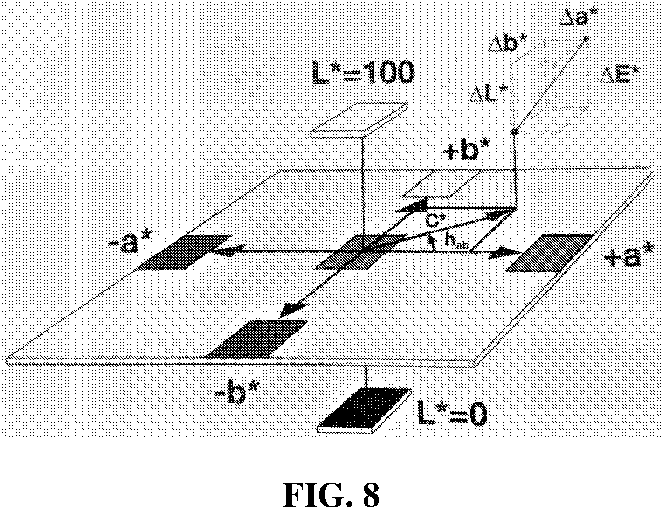

As shown in Figure 8, the use of the CIELab model allows the unambiguous identification of the chosen color within a space defined by the above parameters.

The CIELab model is widely used in industrial practice as it covers the entire spectrum visible to the human eye, representing it uniformly and constitutes an independent model regardless of any graphic technology that may be used by different users. The CIELab values may indeed be easily matched to the simplest representation indexes of the chromatic scales such as RGB and CMYK values, a methodology used by well-known graphic imaging and manipulation software.

According to the ASTM E313 standard in force in 2019, for the characterization of the specific yellow color, a Yellowness Index (YI - Yellowness Index) may be calculated as follows:

$$YI = (100 \, (C_x X - C_z Z)) / Y$$

where X, Y, Z are the values known as CIE tristimulus values, and the coefficients depend on the illuminant and on the observer, as indicated below.

| Coefficients | |
| --- | --- |
| | D65/10 |
| $C_x$ | 1.3013 |
| $C_z$ | 1.1498 |

Further details on the measurement method are indicated in the official ASTM publications concerning the ASTM E313 standard, incorporated herein by reference.

A measurement of the CIELab parameters and the Yellowness Index (YI) was carried out on homogeneous parallelepiped-shaped sample plates, made using the mixture object of the present invention, having the following dimensions:

$$Length = 50 \, mm$$

$$Width = 70 \, mm$$

$$Thickness = 2 \, mm$$

The device used for measuring the CIELab parameters is a spectrophotometer of the type currently known under the trade name CM 3600A from Konica Minolta, but other devices for measuring CIELab parameters may be used to obtain corresponding measurements.

The light radiation used for the measurements is classified as CIE Standard Illuminant D65, according to ISO 11664-2: 2007 (E) / CIE 014-2 S / E: 2006, in force in 2020 using a CIE 1964 standard colorimetric observer placed at an inclination of 10° from a vertical axis perpendicular to the planar development of the plate.

In this condition of measurement, the YI Yellowness Index of a mixture suitable for making a disposable applanation head according to this disclosure was found to be between:

$$76.0 <= YI <= 97.0$$

The CIELab parameters relating to the shade of yellow were found to be included between the following values:

$$92.0\% <= L <= 95.0\%$$

$$-19.0 <= a <= -14.0$$

$$68.0 <= b <= 95.0$$

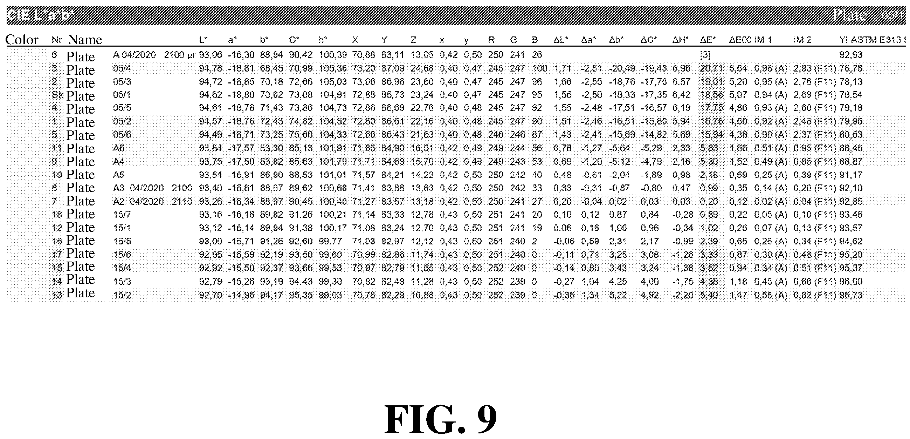

The tables shown in figure 9 report the values of the CIELab and YI parameters measured on sample plates made using suitable transparent yellow mixtures according to the present disclosure.

According to one aspect, the yellow coloring of the portion 5 of the transparent body 8 is identified by means of the CIELab model by means of said parameters L, a, b, where:

 L between 92.0% and 93.4%, preferably 93.0%
 a comprised between -16.6 and -16.0, preferably -16.3
 b comprised between 88.1 and 89.3, preferably 88.94
 and corresponding to a Yellowness Index (YI - Yellowness Index) between 92.1 and 93.4, preferably 92.9.

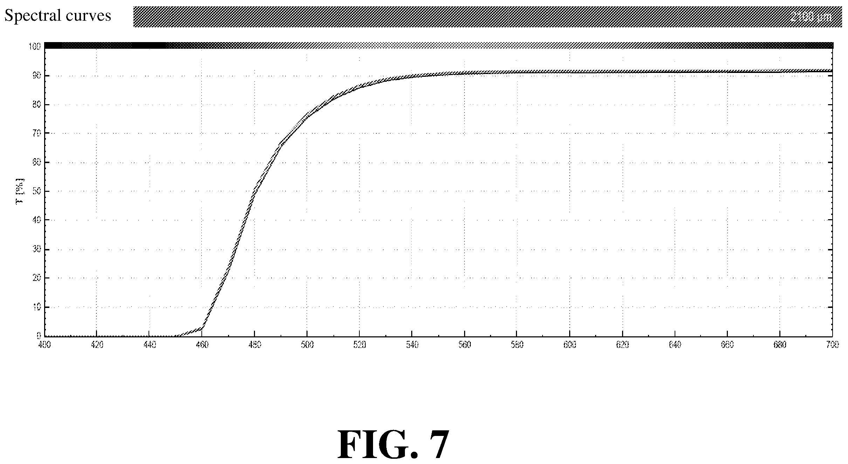

Figure 7 shows the transparency spectrum T (%) of the applanation head 2, made according to a preferred embodiment of the present disclosure, wherein the transparency spectrum is expressed as a percentage and indicates the amount of radiation shielded by the sample, wherein a value T = 0% indicates complete shielding and a value T = 100% indicates complete transparency at the specific wavelength. The diagram shows how the blue radiation, whose spectrum includes wavelengths between 450 and 500nm, reflected by the cornea and covering the entire portion 5 of the applanation head is suitably filtered, and the transparency at longer wavelengths as the green light, 520-570 nm, is almost maximum.

The solution object of the present disclosure is particularly convenient for making disposable applanation heads for Goldmann tonometers. In practice, with the applanation head 2 for a Goldmann tonometer 1 according to this disclosure, it is possible to measure the IOP in the usual way, since the applanation head 2 according to this disclosure can be mounted on the rod of any Goldmann tonometer of a known type, allowing, however, to visualize much better the yellow-green semi-annuli on the blue background.

Conveniently, the applanation head of the present disclosure may be distributed in kit for intraocular pressure measurement with a Goldmann applanation tonometer, which will comprise a sterile package containing an applanation head according to the present disclosure, together with a bottle filled with a liquid dye for ophthalmic use or a strip of paper soaked with a liquid or powder dye for ophthalmic use.

According to one aspect, the bottle will be of the single-dose type and will contain an adequate amount of liquid dye for ophthalmic use, such as fluorescein or riboflavin, to be applied entirely on the corneal surface before carrying out the measurement. Such a kit will provide the doctor with all the consumables necessary to perform a single intraocular pressure measurement and its opening in the presence of each patient will give the patient the certainty that the applanation head used is definitely sterile.

Given that normally two drops of ophthalmic dye are used for each measurement and that the risk of contamination of the ophthalmic dye is extremely low, the kit will conveniently contain multiple individually packaged applanation heads according to this disclosure and a multidose reclosable bottle of ophthalmic dye, which can be a liquid dye, for example based on fluorescein or riboflavin, containing an amount of dye sufficient to perform a number of intraocular pressure measurements corresponding to the number of applanation heads contained in the kit.

Conveniently, the applanation head as well as the bottle will be made of fully recyclable materials.

**Claims**

1. A disposable applanation head (2) for a Goldmann applanation tonometer (1), comprising:
   a transparent body (8) to at least one type of visible light, having a flat corneal applanation surface (6), configured to be pressed against a cornea of an eye so as to flatten it, wherein at least a portion (5) of said transparent body (8) defines optical prisms (9) configured to visualize as two circular semi-annuli a substantially circular perimeter of a cornea flattened against the flat corneal applanation surface (6);

**characterized in that** said transparent body (8) is made of a solidified mixture of transparent polymer with a yellow dye, in which a type and a percentage of said yellow dye of said mixture are determined so that a sample plate, shaped like a parallelepiped, of said solidified mixture having the following dimensions:

$$Length = 50 \text{ mm}$$

$$Width = 70 \text{ mm}$$

$$Thickness = 2 \text{ mm}$$

when this sample plate is illuminated by means of a CIE Illuminant Standard D65 radiation as referred in the ASTM E313-15 standard published on January 1st, 2015, the measurement sensor being a CIE 1964 Standard Colorimetric Observer, as referred in the ASTM E313-15 standard published on January 1st, 2015, positioned at 10° from a vertical axis perpendicular to a planar development of the plate, has a yellow color identified by a CIELab colorimetric model, as referred in the ASTM E313-15 standard published on January 1st, 2015, as a function of parameters L, a, b, wherein:

L between 92.0% and 95.0%
a between -19.0 and -14.0
b between 68.0 and 95.0.

2. The disposable applanation head (2) for a Goldmann applanation tonometer (1) according to claim 1, wherein said yellow coloration of said at least one portion (5) of said transparent body (8) is corresponding to a Yellowness Index (YI - Yellowness Index) in the range of 76.0 to 97.0 when measured according to the ASTM E313-15 standard published on January 1st, 2015.

3. The disposable applanation head (2) for a Goldmann applanation tonometer (1) according to one of the preceding claims, wherein said yellow coloration of said at least one portion (5) of said transparent body (8) is identified by model CIELab by said parameters L, a, b, wherein:

L between 92.0% and 93.4%, preferably 93.0%
a comprised between -16.6 and -16.0, preferably -16.3
b comprised between 88.1 and 89.3, preferably 88.94

and corresponding to a Yellowness Index (YI - Yellowness Index) between 92.1 and 93.4, preferably 92.9.

4. The applanation head (2) for a Goldmann applanation tonometer (1) according to at least one of the preceding claims, wherein said yellow coloration of said at least one portion (5) of said transparent body (8) is obtained by addition of said dye being of polymerization of said transparent polymer in correspondence of said at least one portion (5), in an amount comprised between 0.5% and 1.5% (w / w), preferably 1% (w / w).

5. The applanation head (2) for a Goldmann applanation tonometer (1) according to claim 4, wherein said yellow dye is selected from the group consisting of yellow dyes suitable for coloring PMMA, preferably a yellow dye of the type provided in a concentrated form in a masterbatch using a carrier composed of polymethyl methacrylate (PMMA) or acrylonitrile styrene (SAN).

6. The applanation head (2) for a Goldmann applanation tonometer (1) according to at least one of the preceding claims, wherein said transparent polymer is polymethylmethacrylate (PMMA).

7. The applanation head (2) for a Goldmann applanation tonometer (1) according to at least one of the preceding claims, wherein said head (2) is disposable.

8. An applanation Goldmann tonometer (1), comprising a support rod of an applanation head, a head (2) according to at least one of the preceding claims, and complemented by a slit lamp (3) and relative eyepiece (4).

9. A kit for intraocular pressure measurement with a Goldmann applanation tonometer, comprising a package containing at least one head for Goldmann applanation tonometer (1) according to one of claims 1 to 7, and a bottle filled with a liquid dye for ophthalmic use or at least one strip of paper soaked with a liquid or with a powder dye for ophthalmic use.

**Patentansprüche**

1. Einweg-Applanationskopf (2) für ein Goldmann-Applanationstonometer (1), umfassend:

einen für mindestens eine Art von sichtbarem Licht transparenten Körper (8) mit einer flachen Hornhautapplanationsfläche (6), der so konfiguriert ist, dass er gegen die Hornhaut eines Auges gedrückt wird, um diese abzuflachen, wobei mindestens ein Teil (5) des transparenten Körpers (8) optische Prismen (9) definiert, die so konfiguriert sind, dass sie einen im Wesentlichen kreisförmigen Umfang einer gegen die flache Hornhautapplanationsfläche (6) abgeflachten Hornhaut als zwei kreisförmige Halbringe sichtbar machen;

**dadurch gekennzeichnet, dass** der transparente Körper (8) aus einer verfestigten Mischung aus transparentem Polymer mit einem gelben Farbstoff besteht, wobei eine Art und ein prozentualer Anteil des gelben Farbstoffs in der Mischung so bestimmt sind, dass eine parallelepipedförmige Probenplatte der verfestigten Mischung die folgenden Abmessungen aufweist:

<div align="center">

Länge = 50 mm

Breite = 70 mm

Dicke = 2 mm

</div>

wenn diese Probenplatte mit einer CIE-Lichtart D65 gemäß der am 1. Januar 2015 veröffentlichten Norm ASTM E313-15 beleuchtet wird, wobei der Messsensor ein CIE 1964-Standardfarbmessbeobachter gemäß der am 1. Januar 2015 veröffentlichten Norm ASTM E313-15 ist, 2015, positioniert in einem Winkel von 10° zur vertikalen Achse senkrecht zur ebenen Ausdehnung der Platte, eine gelbe Farbe aufweist, die durch ein CIELab-Farbmesstechnikmodell identifiziert wird, wie in der am 1. Januar 2015 veröffentlichten Norm ASTM E313-15 angegeben, als Funktion der Parameter L, a, b, wobei:

L zwischen 92,0 % und 95,0 %
a zwischen -19,0 und -14,0
b zwischen 68,0 und 95,0 liegt.

2. Einweg-Applanationskopf (2) für ein Goldmann-Applanationstonometer (1) nach Anspruch 1, wobei die gelbe Färbung des mindestens einen Abschnitts (5) des transparenten Körpers (8) einem Gelbindex (YI - Yellowness Index) im Bereich von 76,0 bis 97,0 entspricht, wenn sie gemäß der am 1. Januar 2015 veröffentlichten Norm ASTM E313-15 gemessen wird.

3. Einweg-Applanationskopf (2) für ein Goldmann-Applanationstonometer (1) nach einem der vorhergehenden Ansprüche, wobei die gelbe Färbung des mindestens einen Abschnitts (5) des transparenten Körpers (8) durch das Modell CIELab anhand der Parameter L, a, b identifiziert wird, wobei:

L zwischen 92,0 % und 93,4 %, vorzugsweise bei 93,0 %

a zwischen -16,6 und -16,0, vorzugsweise bei -16,3
b zwischen 88,1 und 89,3, vorzugsweise bei 88,94
und entsprechend einem Gelbindex (YI - Yellowness Index) zwischen 92,1 und 93,4, vorzugsweise bei 92,9 liegt.

4. Applanationskopf (2) für ein Goldmann-Applanationstonometer (1) nach mindestens einem der vorhergehenden Ansprüche, wobei die gelbe Färbung des mindestens einen Abschnitts (5) des transparenten Körpers (8) durch Zugabe des Farbstoffs erreicht wird, der aus der Polymerisation des transparenten Polymers entsprechend dem mindestens einen Abschnitt (5) stammt, in einer Menge von zwischen 0,5 und 1,5 Gew.-%, vorzugsweise 1 Gew.-%.

5. Applanationskopf (2) für ein Goldmann-Applanationstonometer (1) nach Anspruch 4, wobei der gelbe Farbstoff aus der Gruppe ausgewählt ist, die aus zum Färben von PMMA geeigneten gelben Farbstoffen besteht, vorzugsweise ein gelber Farbstoff des Typs, der in konzentrierter Form in einem Masterbatch unter Verwendung eines Trägers aus Polymethylmethacrylat (PMMA) oder Acrylnitrilstyrol (SAN) bereitgestellt wird.

6. Applanationskopf (2) für ein Goldmann-Applanationstonometer (1) nach mindestens einem der vorhergehenden Ansprüche, wobei es sich bei dem transparenten Polymer um Polymethylmethacrylat (PMMA) handelt.

7. Applanationskopf (2) für ein Goldmann-Applanationstonometer (1) nach mindestens einem der vorhergehenden Ansprüche, wobei der Kopf (2) ein Einwegprodukt ist.

8. Applanations-Goldmann-Tonometer (1), das eine Stützstange eines Applanationskopfes, einen Kopf (2) gemäß mindestens einem der vorhergehenden Ansprüche, umfasst und durch eine Spaltlampe (3) und ein entsprechendes Okular (4) ergänzt wird.

9. Kit zur Messung des Augeninnendrucks mit einem Goldmann-Applanationstonometer, umfassend eine Verpackung, die mindestens einen Kopf für ein Goldmann-Applanationstonometer (1) nach einem der Ansprüche 1 bis 7 und eine mit einem flüssigen Farbstoff zur ophthalmologischen Verwendung gefüllte Flasche oder mindestens einen mit einer Flüssigkeit oder einem Pulverfarbstoff zur ophthalmologischen Verwendung getränkten Papierstreifen enthält.

## Revendications

**1.** Tête d'aplanation jetable (2) pour un tonomètre d'aplanation Goldmann (1), comprenant :

un corps transparent (8) à au moins un type de lumière visible, ayant une surface plate d'aplanation de cornée (6), conçue pour être pressée contre une cornée d'un œil afin de l'aplatir, dans laquelle au moins une partie (5) dudit corps transparent (8) définit des prismes optiques (9) conçus pour visualiser sous forme de deux demi-anneaux circulaires un périmètre sensiblement circulaire d'une cornée aplatie contre la surface plate d'aplanation de cornée (6) ; **caractérisée en ce que** ledit corps transparent (8) est constitué d'un mélange solidifié de polymère transparent avec un colorant jaune, dans lequel un type et un pourcentage dudit colorant jaune dudit mélange sont déterminés de sorte qu'une plaque d'échantillon, en forme de parallélépipède, dudit mélange solidifié ayant les dimensions suivantes :

Longueur = 50 mm

Largeur = 70 mm

Épaisseur = 2 mm

lorsque cette plaque échantillon est éclairée au moyen d'un rayonnement CIE Illuminant Standard D65 tel que référencé dans la norme ASTM E313-15 publiée le 1er janvier 2015, le capteur de mesure étant un observateur colorimétrique CIE 1964 Standard, tel que référencé dans la norme ASTM E313-15 publiée le 1er janvier 2015, positionné à 10° d'un axe vertical perpendiculaire à un développement plan de la plaque, a une couleur jaune identifiée par un modèle colorimétrique CIELab, tel que référencé dans la norme ASTM E313-15 publiée le 1er janvier 2015, en fonction des paramètres L, a, b, avec :

L compris entre 92,0 % et 95,0 %
a compris entre -19,0 et -14,0
b compris entre 68,0 et 95,0.

**2.** Tête d'aplanation jetable (2) pour un tonomètre d'aplanation Goldmann (1) selon la revendication 1, dans laquelle ladite coloration jaune de ladite au moins une partie (5) dudit corps transparent (8) correspond à un indice de jaunissement (YI - Indice de jaunissement) compris dans la plage de 76,0 à 97,0 lorsqu'il est mesuré conformément à la norme ASTM E313-15 publiée le 1er janvier 2015.

**3.** Tête d'aplanation jetable (2) pour un tonomètre d'aplanation Goldmann (1) selon l'une des revendications précédentes, dans laquelle ladite coloration jaune de ladite au moins une partie (5) dudit corps transparent (8) est identifiée par le modèle CIELab par lesdits paramètres L, a, b, avec :

L compris entre 92,0 % et 93,4 %, de préférence 93,0 %
a compris entre -16,6 et -16,0, de préférence -16,3
b compris entre 88,1 et 89,3, de préférence 88,94
et correspondant à un indice de jaunissement (YI - Indice de jaunissement) compris entre 92,1 et 93,4, de préférence 92,9.

**4.** Tête d'aplanation (2) pour un tonomètre d'aplanation Goldmann (1) selon au moins l'une des revendications précédentes, dans laquelle ladite coloration jaune de ladite au moins une partie (5) dudit corps transparent (8) est obtenue par addition dudit colorant de polymérisation dudit polymère transparent en correspondance de ladite au moins une partie (5), en une quantité comprise entre 0,5 % et 1,5 % (p/p), de préférence 1 % (p/p).

**5.** Tête d'aplanation (2) pour un tonomètre d'aplanation Goldmann (1) selon la revendication 4, dans laquelle ledit colorant jaune est choisi dans le groupe constitué de colorants jaunes convenant à la coloration du PMMA, de préférence un colorant jaune du type fourni sous forme concentrée dans un mélange-maître à l'aide d'un support composé de polyméthacrylate de méthyle (PMMA) ou d'acrylonitrile styrène (SAN).

**6.** Tête d'aplanation (2) pour un tonomètre d'aplanation Goldmann (1) selon au moins l'une des revendications précédentes, dans laquelle ledit polymère transparent est le polyméthacrylate de méthyle (PMMA).

**7.** Tête d'aplanation (2) pour un tonomètre d'aplanation Goldmann (1) selon au moins l'une des revendications précédentes, dans laquelle ladite tête (2) est jetable.

**8.** Tonomètre d'aplanation Goldmann (1), comprenant une tige de support d'une tête d'aplanation, une tête (2) selon au moins l'une des revendications précédentes, et complétée par une lampe à fente (3) et un oculaire relatif (4).

**9.** Trousse de mesure de la pression intraoculaire avec un tonomètre d'aplanation Goldmann, comprenant un conditionnement contenant au moins une tête pour tonomètre d'aplanation Goldmann (1) selon l'une des revendications 1 à 7, et un flacon rempli d'un colorant liquide à usage ophtalmique ou au moins une bande de papier imbibée d'un liquide ou d'un colorant en poudre à usage ophtalmique.

**Fig. 1**

**Fig. 2**

**Fig. 3**

From the slit lamp to the cornea

Blue light

2

From the cornea to the lens

Yellow-green light

5

**Fig. 4**

a    b    c    d

**Fig. 5**

**Fig. 6**

Spectral curves

**FIG. 7**

**FIG. 8**

| CIE L'a'b' | | | | | | | | | | | | | | | | | | | | | | | | | Plate (6) |
|---|---|---|---|---|---|---|---|---|---|---|---|---|---|---|---|---|---|---|---|---|---|---|---|---|---|
| Color | Nr | Name | | | L' | a* | b* | C* | h* | X | Y | Z | x | y | R | G | B | ΔL* | Δa* | Δb* | ΔC* | ΔH* | ΔE* | ΔE00 IM 1 | IM 2 | YI ASTM E313 |
| | 6 Plate | A 04/2020 | 2100 µr | 93,06 | -16,30 | 88,94 | 90,42 | 100,39 | 70,88 | 83,11 | 13,05 | 0,42 | 0,50 | 250 | 241 | 26 | | | | | | [3] | | | 92,93 |
| | 3 Plate | 05/4 | | 94,78 | -18,81 | 68,45 | 70,99 | 105,36 | 73,20 | 87,09 | 24,68 | 0,40 | 0,47 | 245 | 247 | 100 | 1,71 | -2,51 | -20,49 | -19,43 | 6,96 | 20,71 | 5,64 | 0,96 (A) | 2,93 (F11) | 76,76 |
| | 2 Plate | 05/3 | | 94,72 | -18,85 | 70,18 | 72,66 | 105,03 | 73,06 | 86,96 | 23,60 | 0,40 | 0,47 | 245 | 247 | 96 | 1,66 | -2,55 | -18,76 | -17,76 | 6,57 | 19,01 | 5,20 | 0,95 (A) | 2,76 (F11) | 78,13 |
| St | Plate | 05/1 | | 94,62 | -18,80 | 70,62 | 73,08 | 104,91 | 72,88 | 86,73 | 23,24 | 0,40 | 0,47 | 245 | 247 | 95 | 1,56 | -2,50 | -18,33 | -17,35 | 6,42 | 18,56 | 5,07 | 0,94 (A) | 2,69 (F11) | 78,54 |
| | 4 Plate | 05/5 | | 94,61 | -18,78 | 71,43 | 73,86 | 104,73 | 72,86 | 86,69 | 22,76 | 0,40 | 0,48 | 245 | 247 | 92 | 1,55 | -2,48 | -17,51 | -16,57 | 6,19 | 17,75 | 4,86 | 0,93 (A) | 2,60 (F11) | 79,18 |
| | 1 Plate | 05/2 | | 94,57 | -18,76 | 72,43 | 74,82 | 104,52 | 72,80 | 86,61 | 22,16 | 0,40 | 0,48 | 245 | 247 | 90 | 1,51 | -2,46 | -16,51 | -15,60 | 5,94 | 16,76 | 4,60 | 0,92 (A) | 2,46 (F11) | 79,96 |
| | 5 Plate | 05/6 | | 94,49 | -18,71 | 73,28 | 75,60 | 104,33 | 72,66 | 86,43 | 21,63 | 0,40 | 0,48 | 246 | 246 | 87 | 1,43 | -2,41 | -15,69 | -14,82 | 5,69 | 15,94 | 4,38 | 0,90 (A) | 2,37 (F11) | 80,63 |
| | 11 Plate | A6 | | 93,84 | -17,57 | 83,30 | 85,13 | 101,91 | 71,86 | 84,90 | 16,01 | 0,42 | 0,49 | 249 | 244 | 56 | 0,78 | -1,27 | -5,64 | -5,29 | 2,33 | 5,83 | 1,66 | 0,51 (A) | 0,95 (F11) | 88,46 |
| | 9 Plate | A4 | | 93,75 | -17,50 | 83,82 | 85,63 | 101,79 | 71,71 | 84,69 | 15,70 | 0,42 | 0,49 | 249 | 243 | 53 | 0,69 | -1,20 | -5,12 | -4,79 | 2,16 | 5,30 | 1,52 | 0,49 (A) | 0,85 (F11) | 88,87 |
| | 10 Plate | A5 | | 93,54 | -16,91 | 86,90 | 88,53 | 101,01 | 71,57 | 84,21 | 14,22 | 0,42 | 0,50 | 250 | 242 | 40 | 0,48 | -0,61 | -2,04 | -1,89 | 0,98 | 2,18 | 0,69 | 0,25 (A) | 0,39 (F11) | 91,17 |
| | 8 Plate | A3 04/2020 | 2100 | 93,40 | -16,61 | 88,67 | 89,62 | 100,68 | 71,41 | 83,88 | 13,63 | 0,42 | 0,50 | 250 | 242 | 33 | 0,33 | -0,31 | -0,87 | -0,80 | 0,47 | 0,99 | 0,35 | 0,14 (A) | 0,20 (F11) | 92,10 |
| | 7 Plate | A2 04/2020 | 2110 | 93,26 | -16,34 | 88,97 | 90,45 | 100,40 | 71,27 | 83,57 | 13,18 | 0,42 | 0,50 | 250 | 241 | 27 | 0,20 | -0,04 | 0,02 | 0,03 | 0,03 | 0,20 | 0,12 | 0,02 (A) | 0,04 (F11) | 92,85 |
| | 18 Plate | 15/7 | | 93,16 | -16,18 | 89,82 | 91,26 | 100,21 | 71,14 | 83,33 | 12,78 | 0,43 | 0,50 | 251 | 241 | 20 | 0,10 | 0,12 | 0,87 | 0,84 | -0,28 | 0,89 | 0,22 | 0,05 (A) | 0,10 (F11) | 93,46 |
| | 12 Plate | 15/1 | | 93,12 | -16,14 | 89,94 | 91,38 | 100,17 | 71,08 | 83,24 | 12,70 | 0,43 | 0,50 | 251 | 241 | 19 | 0,06 | 0,16 | 1,00 | 0,96 | -0,34 | 1,02 | 0,26 | 0,07 (A) | 0,13 (F11) | 93,57 |
| | 16 Plate | 15/5 | | 93,00 | -15,71 | 91,26 | 92,60 | 99,77 | 71,03 | 82,97 | 12,12 | 0,43 | 0,50 | 251 | 240 | 2 | -0,06 | 0,59 | 2,31 | 2,17 | -0,99 | 2,39 | 0,65 | 0,26 (A) | 0,34 (F11) | 94,62 |
| | 17 Plate | 15/6 | | 92,95 | -15,59 | 92,19 | 93,50 | 99,60 | 70,99 | 82,86 | 11,74 | 0,43 | 0,50 | 251 | 240 | 0 | -0,11 | 0,71 | 3,25 | 3,08 | -1,26 | 3,33 | 0,87 | 0,30 (A) | 0,48 (F11) | 95,20 |
| | 15 Plate | 15/4 | | 92,92 | -15,50 | 92,37 | 93,66 | 99,53 | 70,97 | 82,79 | 11,55 | 0,43 | 0,50 | 252 | 240 | 0 | -0,14 | 0,80 | 3,43 | 3,24 | -1,38 | 3,52 | 0,94 | 0,34 (A) | 0,51 (F11) | 95,37 |
| | 14 Plate | 15/3 | | 92,79 | -15,26 | 93,19 | 94,43 | 99,30 | 70,82 | 82,49 | 11,28 | 0,43 | 0,50 | 252 | 239 | 0 | -0,27 | 1,04 | 4,25 | 4,00 | -1,75 | 4,38 | 1,18 | 0,45 (A) | 0,66 (F11) | 96,00 |
| | 13 Plate | 15/2 | | 92,70 | -14,96 | 94,17 | 95,35 | 99,03 | 70,78 | 82,29 | 10,88 | 0,43 | 0,50 | 252 | 239 | 0 | -0,36 | 1,34 | 5,22 | 4,92 | -2,20 | 5,40 | 1,47 | 0,56 (A) | 0,82 (F11) | 96,73 |

# FIG. 9

**REFERENCES CITED IN THE DESCRIPTION**

**Patent documents cited in the description**

- EP 3260041 A